# EUROPEAN PATENT APPLICATION

(11) **EP 4 122 536 A1**
(43) Date of publication of application: **25.01.2023**
(21) Application number: 21771565.5
(22) Date of filing: 15.03.2021
(51) Int. Cl.: A61P 3/00, A61P 39/06, A23L 33/13, A61K 31/706

(54) **COENZYME Q PRODUCTION PROMOTER AND COENZYME Q PRODUCTION PROMOTING METHOD**

(30) Priority: 16.03.2020 JP 2020044908
(71) Applicant: Tanaka, Megumi, Chigasaki-shi, Kanagawa 253-0054 (JP); Tanaka, Tsunemaru, Chigasaki-shi, Kanagawa 256-0815 (JP)
(72) Inventor: SETOYAMA Daiki, Fukuoka-shi, Fukuoka 812-8582 (JP); KANG Dongchon, Fukuoka-shi, Fukuoka 812-8582 (JP); TANAKA Megumi, Chigasaki-shi, Kanagawa 253-0054 (JP); TANAKA Tsunemaru, Chigasaki-shi, Kanagawa 256-0815 (JP)
(74) Representative: IPAZ
(86) International application number: PCT/JP2021/010274
(87) International publication number: WO 2021/187396

(57) **Abstract**

[PROBLEM] To provide a coenzyme Q production accelerator that allows increasing an intracellular coenzyme Q level.

[SOLUTION] The present invention is a coenzyme Q production accelerator containing nicotinamide mononucleotide as an active ingredient. [SELECTED DRAWING] None

## Description

### [TECHNICAL FIELD]

The present invention relates to a coenzyme Q production accelerator and a method for accelerating coenzyme Q production.

### [BACKGROUND ART]

Coenzyme Q is a lipid-soluble in vivo substance that is biosynthesized in almost every organism starting with human. In a eucaryote, coenzyme Q exists the most in inner membranes of mitochondria, and is an indispensable structural component of the electron transport system in which coenzyme Q functions as a carrier for electron transportation in energy (ATP) production caused by oxidative phosphorylation. The structure of coenzyme Q is constituted of a benzoquinone ring and an isoprenoid side chain, and types of coenzyme Q with different chain lengths exist in the natural world. For example, the main coenzyme Q of a human, cattle, and the like is coenzyme Q10 in which the isoprenoid side chain has 10 units, the main coenzyme Q of a mouse, a rat, and the like is coenzyme Q9, and the main coenzyme Q of lower organisms is coenzyme Q6 to 9. Coenzyme Q10 exists in three states, an "oxidized form" (ubiquinone), a "radical intermediate form" (semiquinone radical), and a "reduced form" (ubiquinol), and the ubiquinone becomes the ubiquinol by undergoing a two-electron reduction. It is considered that 60% to 80% of coenzyme Q10 in a human body exists in the reduced form. The structure of coenzyme Q10 is described below.

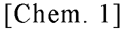

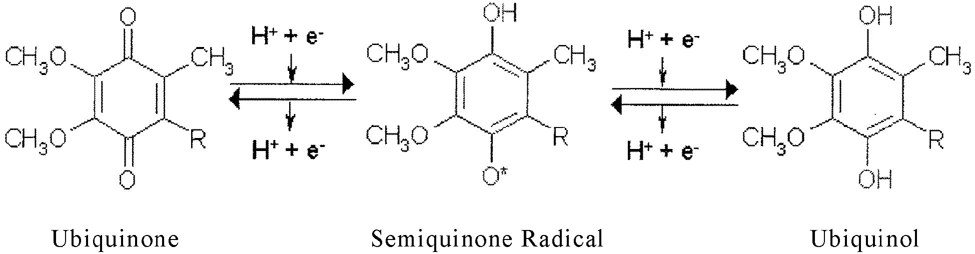

As a physiological effect of coenzyme Q (especially in the reduced form), a function of increasing cellular energy production and decreasing physical fatigue caused in ordinary life (energy metabolism improving effect) has been reported. Therefore, in a human body, high levels of coenzyme Q10 are found in organs with active energy metabolism and having high demands of energy. Typically, the heart, brain, kidney, liver, spleen, pancreas, and the like include coenzyme Q10 with relatively high concentration.

Further, coenzyme Q is known to have an antioxidant effect in addition to the above-described energy metabolism improving effect. Since modern life causes exposure to various kinds of stress, an inside of a living body is under a condition where reactive oxygen is easily generated. Coenzyme Q as an endogenously produced oxidation inhibitor constitutes one of in vivo systems for removing the reactive oxygen. That is, coenzyme Q shows an ability to function as an antioxidant substance by repeating a redox reaction, and exhibits a high defensive effect against various kinds of oxidative stress in vivo that are considered to be causes of aging and various illnesses. Especially in human, ubiquinol as the reduced form of coenzyme Q10 exhibits a high antioxidant effect and is believed to be effective for preventing lifestyle-related diseases, enhancing the immune system, and the like by suppressing cellular damage caused by reactive oxygen. As described above, coenzyme Q is an important molecule in vivo related to important physiological effects for vital activity, namely, the energy metabolism improving effect and the antioxidant effect.

Although coenzyme Q10 needed in a human body is secured by being replenished through in vivo production, meals, and the like, an amount of coenzyme Q10 synthesized in vivo decreases with age starting from approximately age 20, and this decrease is related to various dysfunctions accompanied by aging. With age, the contained amount of coenzyme Q10 decreases in almost every organ. For example, in a mouse, organs in which coenzyme Q10 significantly decreases are the heart, kidney, and spleen, in which energy metabolism actively occurs. Particularly, the heart, which continuously beats, requires a large amount of energy and thus affected greatly by the decrease of coenzyme Q10.

Further, the decrease of coenzyme Q10 is induced not only by aging, but also by medicine intake and diseases. As one example, ingestion of statins (HMG-CoA reductase inhibitors) as therapeutic drugs for hyperlipidemia (hyperlipemia), which have an effect of decreasing LDL-C (LDL cholesterol), is known to decrease the coenzyme Q10 level in vivo. For example, a case has been reported where 20 mg/day of pravastatin, simvastatin, and the like were taken for 12 weeks, resulting in an approximately 50% decrease of coenzyme Q10 amount in the blood.

Further, as another example of the above-described diseases, in mitochondrial diseases caused by mitochondria dysfunction, biosynthesis disorders of coenzyme Q10 and the like occur, and as a result, various symptoms are induced. Conversely, mutations of a gene cluster related to coenzyme Q10 biosynthesis induce fatal multiple organ failure, central nervous system disorders, and the like. The decrease in coenzyme Q10 amount caused by mitochondria dysfunction is related to various diseases. For example, several subtypes of heart disease are suggested to be possibly induced by a decrease in coenzyme Q10 amount in heart muscle mitochondria. In actuality, it is pointed out that a blood coenzyme Q10 level of a heart failure patient tends to decrease.

While a healthy human can obtain sufficient coenzyme Q10 for preventing coenzyme Q10 deficiency by daily meals and in vivo synthesis, as mentioned above, with a decrease in the coenzyme Q10 production level caused by aging, medicine intake, and the like, securing a necessary and sufficient amount of coenzyme Q10 becomes difficult. Therefore, to replenish the lack of coenzyme Q10, coenzyme Q10 is extrinsically replenished as a medicinal product, food product, or a supplement.

In this country, in the pharmaceutical field, coenzyme Q10 (oxidized form) is listed in the Japanese Pharmacopoeia under the general name: Ubidecarenone. As a medicinal product, ubidecarenone is used as a medicine for improving myocardial metabolism and, intended for efficacy and effect on a "light to medium degree congestive heart failure symptom undergoing basic treatment," is approved to be taken 30 mg per day. Ubidecarenone is confirmed to be absorbed through the lymphatic vessels by oral administration, move to the intracellular mitochondria, and activate ATP production in the intracellular electron transport system. In addition, pharmacologically, ubidecarenone is recognized to keep oxygen shortage in the heart muscle induced by isoprenaline at a light degree. Further, ubidecarenone is recognized to improve heart failure caused by high blood pressure and the like. Clinically, it is confirmed by a double-blind comparative test that, for congestive heart failure with a heart disease, such as ischemic heart disease, valvular disease, and cardiomyopathy, as a pre-existing condition, by administering the present agent in addition to conventional therapeutic drugs (such as digitalis-diuretic), congestive heart failure symptoms are improved. For such improvement of heart disease, substitution therapies (oral supplement and vein injection) of coenzyme Q10 have been performed at a global level, and effects to some extent have been recognized.

Further, coenzyme Q10, which has been used as a medicinal product component, was approved to be used as a food component in year 2001, and since then, various health food products (supplements) have been sold. As health food products, products with a daily recommended amount of 60 mg are distributed the most, and products with a daily recommended amount of over 30 mg account for 80% or more of the entire products, while a product with a daily recommended amount of 300 mg is also distributed.

As above, attempts are made to treat and prevent various diseases caused by coenzyme Q10 deficiency by extrinsically supplying exogenous coenzyme Q10. For example, PLT 1 reports a food composition for decreasing blood LDL cholesterol concentration, for decreasing blood LDL cholesterol concentration and fatigue recovery, or for fatigue recovery, containing coenzyme Q10 of 1.8 times or more the standard intake of coenzyme Q10 as a substitution of a medicinal product for preventing progress of lifestyle-related diseases.

As another example, PLT 2 reports an antioxidant supplement as an antioxidant supplement formulated with the reduced form of coenzyme Q10, which works more effectively in vivo, instead of the oxidized form of coenzyme Q10, which is conventionally often used, and containing vitamin E, vitamin C, the reduced form of coenzyme Q10, α-lipoic acid, anthocyanidin, β-carotene, selenium, S-adenosylmethionine, vitamin B6, vitamin B12, and folic acid.

### [PRIOR ART DOCUMENT]

### [PATENT DOCUMENT]

PLT 1: JP-A-2018-14912
PLT 2: JP-A-2015-218140

### [DISCLOSURE OF THE INVENTION]

### [PROBLEMS TO BE SOLVED BY THE INVENTION]

However, the prior art of extrinsically replenishing exogenous coenzyme Q10 as a supplement and the like is considered to provide an improving effect using the antioxidant effect of coenzyme Q10, rather than coenzyme Q10 being directly taken into organs and providing a function of accelerating intracellular energy metabolism.

Further, since extrinsically replenished coenzyme Q10 has a considerably high lipophilicity, thus a large portion being insoluble to water, and has a relatively large molecular weight, an absorption efficiency of orally administered coenzyme Q10 is low. Therefore, it is questionable whether the effects of coenzyme Q10 can be sufficiently expected in extrinsically replenished coenzyme Q10.

Further, in a case where coenzyme Q10 is ingested, since the absorption rate is different for each organ and almost none of the coenzyme Q10 gets absorbed especially in organs that require coenzyme Q10 the most, such as the heart and kidney, there has been a problem that, by only ingesting exogenous coenzyme Q10, it is difficult to replenish a required amount of coenzyme Q10 in organs with high demands of energy that require coenzyme Q10 the most, such as the heart and kidney.

Therefore, it is an object of the present invention to provide a coenzyme Q production accelerator that can increase the level of intracellular endogenous (biosynthetically derived) coenzyme Q.

### [SOLUTIONS TO THE PROBLEMS]

As a result of serious study for solving the above-described problems, the inventor discovered for the first time in the world that nicotinamide mononucleotide, which is an intermediate metabolite related to biosynthesis of coenzyme nicotinamide adenine dinucleotide (NAD), accelerates coenzyme Q production, and based on this breakthrough research result, deemed that nicotinamide mononucleotide can be applied to, for example, preventing or treating diseases derived from coenzyme Q deficiency. Thus, the present invention was completed.

The present invention is as follows.
[1] A coenzyme Q production accelerator that contains nicotinamide mononucleotide as an active ingredient.
[2] The coenzyme Q production accelerator according to [1] where coenzyme Q is coenzyme Q10 or coenzyme Q9.
[3] The coenzyme Q production accelerator according to [1] or [2] where coenzyme Q exists in a heart, brain, kidney, liver, spleen, or pancreas.
[4] A food and beverage product for accelerating coenzyme Q production where the food and beverage product contains the coenzyme Q production accelerator according to [1].
[5] A medicinal product for treating or preventing a disease caused by coenzyme Q deficiency where the medicinal product contains the coenzyme Q production accelerator according to [1].
[6] A method for accelerating coenzyme Q production that includes causing a target to ingest an effective dose of the coenzyme Q production accelerator according to [1] .

### [EFFECTS OF THE INVENTION]

The present invention can activate coenzyme Q biosynthesis in mitochondria inside an organ and increase the level of intracellular endogenous coenzyme Q, and thus, able to directly increase the coenzyme Q level even in the heart, kidney, and the like, where exogenous coenzyme Q is difficult to be absorbed. In addition, the level of the reduced form of coenzyme Q, which has been difficult to be supplied by the conventional exogenous coenzyme Q, can also be increased. Therefore, the present invention is highly effective for preventing or treating diseases caused by coenzyme Q deficiency. In addition, since nicotinamide mononucleotide, which is an intermediate metabolite related to in vivo NAD⁺ biosynthesis, is contained as an active ingredient, the present invention is safe and can be ingested over a long period of time.

### [BRIEF DESCRIPTION OF THE DRAWINGS]

Fig. 1a is a graph diagram indicating results of Embodiment 1.
Fig. 1b is another graph diagram indicating results of Embodiment 1.
Fig. 2 is an explanatory diagram illustrating metabolic pathways related to niacin (general term for nicotinamide and nicotinic acid).

### [DESCRIPTION OF PREFERRED EMBODIMENTS]

A coenzyme Q production accelerator according to the present invention (hereinafter may be referred to as "the present agent") contains nicotinamide mononucleotide as an active ingredient, activates coenzyme Q biosynthesis in mitochondria, and accelerates coenzyme Q production. While an action mechanism of the present agent has not been entirely solved, it is suspected that nicotinamide mononucleotide increases the amount of nicotinamide adenine dinucleotide (NAD) and accelerates a catalytic reaction of sirtuins, while activating mitochondria and increasing ATP production to accelerate coenzyme Q production. Accordingly, the present agent is considered to provide an effect of preventing or treating diseases caused by coenzyme Q deficiency by causing nicotinamide mononucleotide as an active ingredient to increase the level of coenzyme Q.

The present agent activates coenzyme Q production using nicotinamide mononucleotide as an active ingredient to increase the level of endogenous coenzyme Q10, and thus allows a physiological effect of coenzyme Q10 to be more efficiently and effectively provided than exogenous coenzyme Q10. Therefore, by ingesting nicotinamide mononucleotide as an active ingredient instead of exogenous coenzyme Q10, for diseases induced by a decrease in the coenzyme Q10 level (the most major one being heart disease), a symptom improving effect can be expected in a short term or, by preliminary continuing ingestion as a supplement and the like, a disease prevention effect can be obtained. For example, by causing a subject with a heart disease caused by a decreased level of coenzyme Q10 to ingest the present agent, an effect equivalent to that of supplementing coenzyme Q10 directly to mitochondria in the heart can be expected.

The present agent activates coenzyme Q production in various organs, especially the heart, brain (especially the cerebellum), kidney, liver, spleen, and pancreas to increase the level of endogenous coenzyme Q10 using nicotinamide mononucleotide as an active ingredient.

The nicotinamide mononucleotide (chemical formula: C₁₁H₁₅N₂O₈P) is a compound produced in bodies of many organisms including human, and expressed with a structural formula [Chem. 2] below. The nicotinamide mononucleotide is generally referred to as NMN, and known as an intermediate metabolite involved in a biosynthesis of coenzyme NAD⁺.

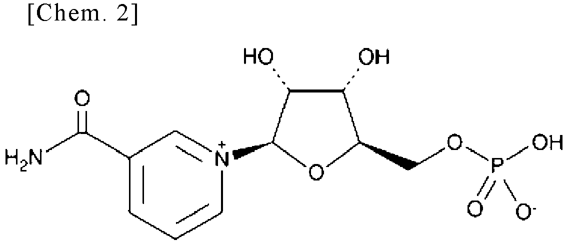

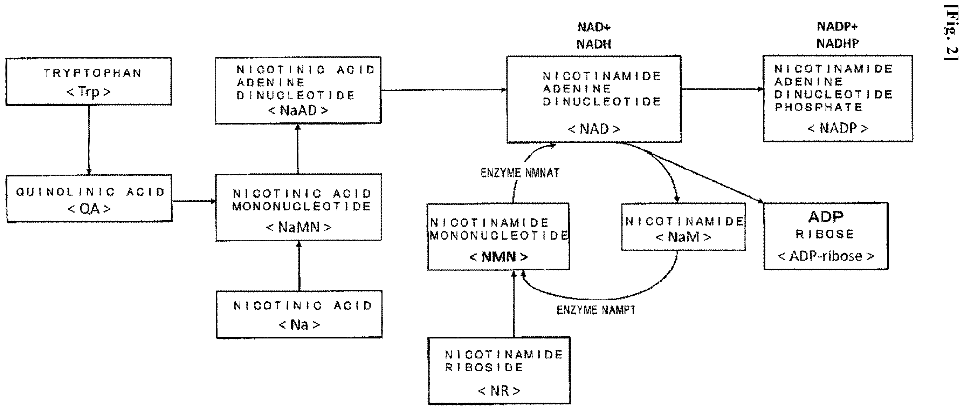

The nicotinamide mononucleotide is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to Fig. 2. Fig. 2 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

As illustrated in Fig. 2, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN). Meanwhile, when the nicotinic acid (Na) is the starting material, the nicotinic acid is directly converted into the NaMN. Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD. Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730). Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available from Oriental Yeast Co., ltd. and Bontac Bio-engineering (Shenzhen) Co., Ltd., and those commercial products can be purchased for use.

The nicotinamide mononucleotide is a purified product that contains a few impurities, especially, preferably its purity is 90% or more, and further preferably its purity is 95% or more. When the purity is less than 90%, a bad smell possibly occurs, or the effect of the nicotinamide mononucleotide is possibly reduced to fail to sufficiently provide the effect of the present invention.

The present agent can be easily manufactured by using nicotinamide mononucleotide alone or by mixing in other components. The other components are not specifically limited as long as the present agent provides the effect of the present invention.

Examples of other components include, as described above, carotenoid (such as lycopene, lutein, and vitamin A), astaxanthin, polyphenols (such as flavonoid, catechin, isoflavone, sesamin, and curcumin), vitamin E, and vitamin C, which are known to be food components that remove reactive oxygen; vitamin A, vitamin E, vitamin C, and zinc, which are known to be food components that enhance immunity; and docosahexaenoic acid (DHA), eicosapentaenoic acid (EPA), lecithin, tryptophan, which are known to be food components that suppress aging; and the like. In addition, for example, various vitamins, trace amounts of elements, citric acid, malic acid, flavoring agents, inorganic salt, and the like, which are common supplemental components in the food product field can be included as other components.

To provide the effect of the present invention, the present agent intends the amount of nicotinamide mononucleotide as an active ingredient to be a sufficient amount for activating mitochondria and accelerating coenzyme Q production. The specific amount is different depending on, for example, the age, sex, and weight of a target that takes the present agent, the expected effect, and the symptom, but the daily intake per adult of the nicotinamide mononucleotide is ordinarily 1 mg to 500 mg, preferably 5 mg to 350 mg, and more preferably 50 mg to 300 mg, and further more preferably 150 mg to 300 mg. Less than 1 mg possibly fails to provide the effect of the present invention, while more than 500 mg merely provides almost similar effect but causes economic disadvantage.

The present agent can be used for objects of accelerating coenzyme Q production and treating or preventing diseases caused by coenzyme Q deficiency. The diseases caused by coenzyme Q deficiency include mitochondrial diseases (such as mitochondrial encephalomyopathy), lifestyle-related diseases, cardiovascular diseases (such as congestive heart failure, myocardial infarction, angina pectoris, and vascular endothelial dysfunction), diabetes, and neurodegenerative diseases (such as Parkinson's disease, Huntington's disease, and Friedreich's ataxia) caused by coenzyme Q deficiency. Note that, in the present invention, "treating diseases caused by coenzyme Q deficiency" means curing a certain disease or mitigating symptoms. This includes, for example, stopping progress, downstaging, and suppressing metastasis of the disease. In addition, "preventing diseases caused by coenzyme Q deficiency" means not only preventing onset of a certain disease but also broadly includes preventing recurrence/relapse and exacerbation after the illness has cured or mitigated.

A method for manufacturing the present agent is not specifically limited, and a common manufacturing method applied for manufacturing the present agent according to its form may be selected as appropriate. For example, in a case of a powder form, the present agent can be manufactured by uniformly mixing nicotinamide mononucleotide and other components added in as necessary. Note that, nicotinamide mononucleotide as an active ingredient is distributed in the market and is commercially available. Especially, in recent years, a quality management system and a mass production system of nicotinamide mononucleotide have been established.

The present agent can be used as a food and beverage product, a medicinal product, and the like. In a case of being used as a food and beverage product, the present agent can be provided as a food and beverage product for accelerating coenzyme Q production in the food product field. By daily ingesting the present agent in the form of a food product, the effect of the present invention can be provided repeatedly and is thus especially effectual for enjoying the effect even better. The type of the food product as the target of the present invention is not specifically limited, and the target includes a functional food, a food for specified health use, a dietary supplement, a food additive, a feed, a care food, a diet therapy food, a therapeutic diet, a diet food, and similar food product in addition to general food products. Specifically, for example, confectionery (gum, candies, cookies, gummi candies, biscuits, cakes, chocolates, Japanese confectionery, jelly, and the like), bread, noodles, rice/grain processed foods (cereals and the like), meat processed foods, fish and shellfish processed foods, vegetable processed foods, ready-prepared foods, fermented foods, seasonings (source, dressing, ketchup, and the like), spices, dairy products (yogurt, cheese, milk, and the like), ice cream, frozen foods, retort pouch foods, beverages (carbonated beverages, soft drinks, milk-based beverages, alcoholic beverages, sports beverages, fruit-flavored beverages, teas, nutritious beverages, concentrated beverages, and the like), powdered beverages (powdered juice, powdered soup, and the like) are exemplified. The form of the food product is not limited, and especially in the case of the functional food, the food for specified health use, and the like, the food product can be processed to be provided in the form of, for example, a powder, a tablet, a pill, a granule, a hard capsule formulation, a soft capsule formulation, a jelly, a liquid medicine, and a paste medicine.

The intake of the food product is different depending on the type of the food product, age, sex, and weight of a target that takes the food product, the expected effect, and the symptom. However, the daily intake per adult of the nicotinamide mononucleotide contained in the food product is ordinarily 1 mg to 500 mg, preferably 5 mg to 350 mg, more preferably 50 mg to 300 mg, and further more preferably 150 mg to 300 mg. Less than 1 mg possibly fails to provide the effect of the present invention, while more than 500 mg merely provides almost similar effect but causes economic disadvantage. The compounding ratio of the nicotinamide mononucleotide in the food product can be appropriately set relative to a total food weight in a range of 100% or less.

The food product is safe and side effects are not specifically recognized. Therefore, the food product can be ingested over a long period of time. The food product can be applied to not only the elderly people but also the young people.

On the other hand, in the pharmaceutical field, the present agent can be administered orally or not orally as a medicinal product (including a quasi-drug) with an object to treat or prevent diseases caused by coenzyme Q deficiency. A dosage form of the medicinal product is not specifically limited, but can include, for example, a powder, a tablet, a persistent tablet, a chewable tablet, an effervescent tablet, a troche, a buccal tablet, a sublingual tablet, a capsule formulation, a fine granule, a granule, a pill, a dry syrup, a liquid medicine, a suspending agent, a syrup, a formulation for oral administration such as an elixir, and an eye drop, an eyewash, an eye ointment, an injection preparation, a transfusion, and an external preparation. Among these forms, considering the ease of taking, the stability of the active ingredient, and the like, the formulation for oral administration such as the powder, the tablet, and the capsule formulation is preferable. Diseases caused by coenzyme Q deficiency are as already mentioned in the description of the present agent.

The medicinal product can appropriately contain a known additive for formulation, which is adequate for the dosage form and pharmacologically allowed, considering physicochemical property, biological property, and similar property. Such an additive for formulation is exemplified by, for example, an excipient (lactose, starch, crystalline cellulose, sodium phosphate, and the like), a solvent (water, soybean oil, saline solution, a nonaqueous solvent for injection, and the like), a binder (starch, gelatin, gum arabic, sodium alginate, carmellose sodium, methylcellulose, ethyl cellulose, hydroxypropyl methylcellulose, hydroxypropyl cellulose, polyvinylpyrrolidone, and the like), a disintegrant (starch, carmellose sodium, and the like), a lubricant (talc, magnesium stearate, calcium stearate, macrogol, sucrose fatty acid ester, and the like), a coating agent (white sugar, HPC, shellac, gelatin, glycerin, hydroxypropyl methylcellulose, hydroxypropyl methylcellulose phthalate, cellulose acetate phthalate, and the like), a stabilizer (sodium bisulfite, sodium thiosulfate, sodium edetate, sodium citrate, ascorbic acid, dibutylhydroxytoluene, and the like), a preservative (methyl parahydroxybenzoate, ethyl parahydroxybenzoate, propyl parahydroxybenzoate, benzyl alcohol, phenol, chlorobutanol, benzalkonium chloride, benzethonium chloride, sodium dehydroacetate, thimerosal, and the like), a viscous agent (methylcellulose, carmellose sodium, chondroitin sulfate, sodium alginate, and the like), a suspending agent (various nonionic surfactant, methylcellulose, carmellose sodium, and the like), an emulsifier (gum arabic, cholesterol, sorbitan sesquioleate, polysorbate 80, sodium lauryl sulfate, and the like), a buffer (citric acid, acetic acid, sodium phosphate, and boric acid), a surfactant (hydrogenated castor oil, polysorbate 80, and the like), a colorant (water-soluble food pigment, lake pigment, and the like), a corrigent (lactose, white sugar, glucose, mannitol, and the like), a scenting agent (aromatic essential oils), a plasticizer (the phthalic acid esters, vegetable oils, polyethylene glycol, and the like).

A dose of the medicinal product cannot be equally specified because it differs depending on, for example, age, weight, and symptom of the administration target and the number of times of the administration. However, as the dose of the medicinal product, the daily amount per adult of the nicotinamide mononucleotide to be administered is ordinarily 1 mg to 500 mg, preferably 5 mg to 350 mg, and more preferably 50 mg to 300 mg, and further more preferably 150 mg to 300 mg. Less than 1 mg possibly fails to provide the effect of the present invention, while more than 500 mg merely provides almost similar effect but causes economic disadvantage. The compounding ratio of the nicotinamide mononucleotide in the medicinal product can be appropriately set in accordance with the dosage form, the dose of the medicinal product, and the like.

The number of times of the administration of the medicinal product can be appropriately set in accordance with, for example, the age, weight, and symptom of the administration target and the dose per administration of the medicinal product. The number of times of the medicinal product administration per day can be exemplified by once to three times.

As described above, by administering an amount of nicotinamide mononucleotide sufficient for accelerating coenzyme Q production, an effect of treating or preventing diseases caused by coenzyme Q deficiency can be provided. Thus, the present invention further provides a method that includes causing a target needing the present agent to ingest an effective dose (that is, the above-mentioned amount) of the present agent, and treating or preventing diseases caused by coenzyme Q deficiency by activating mitochondria and accelerating coenzyme Q production. The target of the ingestion is preferably a mammal such as a human, a mouse, a rat, a rabbit, a dog, a cat, cattle, a horse, a pig, and a monkey, and especially a human is preferable. In the method, the intake of the nicotinamide mononucleotide, the number of intake per day, and similar matters are as described for the agent. The a present agent can be ingested anytime in any case, and can be ingested by the target over a long period of time.

### [Working Example]

The following describes the present invention in detail based on the working examples, while the present invention is not limited by these working examples.

### (Working Example 1)

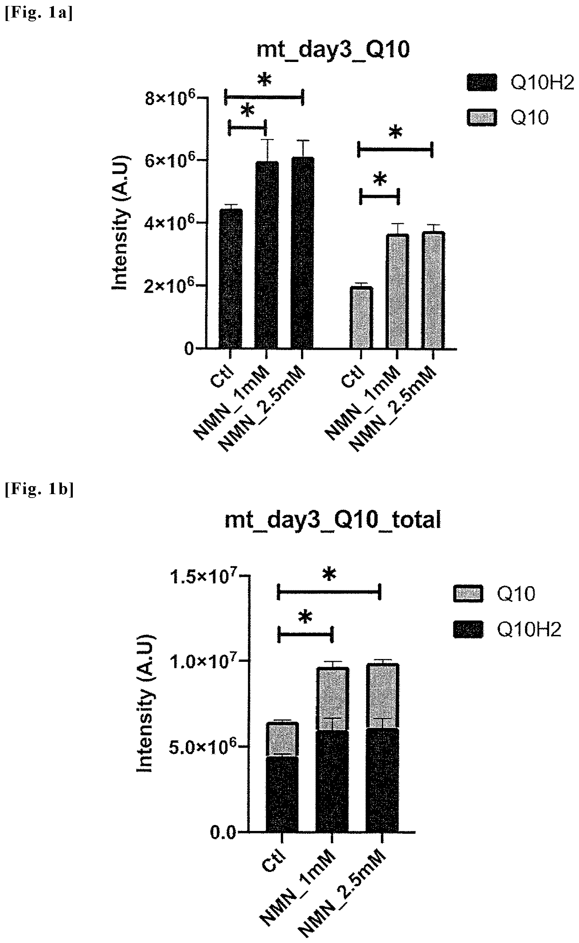

To examine a production promoting effect of coenzyme Q10 caused by nicotinamide mononucleotide, HEK293 cells (human embryonic kidney cells) were cultured for three days (37°C, 5% CO₂) in DMEM medium containing β-nicotinamide mononucleotide with a predetermined concentration (0 mM (control), 1 mM, and 2.5 mM daily added). Three days after the first day of culture, mitochondria were isolated from the HEK293 cells, and contained amounts of each of the reduced form (ubiquinol) and the oxidized form (ubiquinone) of coenzyme Q10 in the mitochondria were quantified by LC-MS method (LC column: KINETEX C18 column (Phenomenex), MS: Shimadzu LCMS-8060 (Shimadzu Corporation), eluent: 10 mM ammonium formate in Methanol, flow rate: 0.5 mL/minute, detection: MS/MS analysis by multiple reaction monitoring (MRM) method; Q10H2 883.6 -> 197.15 m/z, Q10 881.4 -> 197.15 m/z, Q9H2 815.6 -> 197.15 m/z, Q9 813.6 -> 197.15 m/z, Q8H2 747.6 -> 197.15 m/z, Q8 745.6 -> 197.15 m/z) (column: ODS, column temperature: 40°C, eluent: 70% acetonitrile/methanol (1:1) and 30% 0.05 M ammonium formate (prepared to be pH 7.2 with 10% ammonia water), flow rate: 1.0 mL/minute, detection: UV276 nm) (column: ODS, column temperature: 40°C, eluent: 70% acetonitrile/methanol (1:1) and 30% 0.05 M ammonium formate (prepared to be pH 7.2 with 10% ammonia water), flow rate: 1.0 mL/minute, detection: UV276 nm). The results are shown in Figs. 1a, b. Fig. 1a shows respective changes of the contained amounts of Q10H2 and Q10, and Fig. 1b shows a change of the sum amount of Q10H2 and Q10. Note that, in Figs. 1a, b, Q10H2 indicates the reduced form of coenzyme Q10 (ubiquinol) and Q10 indicates the oxidized form of coenzyme Q10 (ubiquinone). In addition, "*" indicates that a p-value in a student's two-sided t-test is less than 0.05.

As a result, it was discovered that, in a medium containing nicotinamide mononucleotide, the contained amounts of the reduced form and the oxidized form of coenzyme Q10 were each increased approximately by 1.5 times to being double. Further, it was discovered that the sum amount of the reduced form and the oxidized form of coenzyme Q10 also approximately doubled. The result indicates that, in the cells provided with nicotinamide mononucleotide, coenzyme Q10 (in both the reduced form and the oxidized form) production in the mitochondria was activated, and an achievement of a significant increase of coenzyme Q10 was confirmed.

## Claims

1. A coenzyme Q production accelerator that contains nicotinamide mononucleotide as an active ingredient.

2. The coenzyme Q production accelerator according to claim 1, wherein coenzyme Q is coenzyme Q10 or coenzyme Q9.

3. The coenzyme Q production accelerator according to claim 1 or 2, wherein coenzyme Q exists in a heart, brain, kidney, liver, spleen, or pancreas.

4. A food and beverage product for accelerating coenzyme Q production, wherein
the food and beverage product contains the coenzyme Q production accelerator according to claim 1.

5. A medicinal product for treating or preventing a disease caused by coenzyme Q deficiency, wherein
the medicinal product contains the coenzyme Q production accelerator according to claim 1.

6. A method for accelerating coenzyme Q production, comprising
causing a target to ingest an effective dose of the coenzyme Q production accelerator according to claim 1.
